# EUROPEAN PATENT APPLICATION

(11) **EP 4 043 023 A1**
(43) Date of publication of application: **17.08.2022**
(21) Application number: 20870206.8
(22) Date of filing: 09.09.2020
(51) Int. Cl.: A61K 36/53, A61K 8/9789, A61K 8/99, A61K 35/747, A61P 17/00, A61P 43/00, A61Q 19/00, A61Q 19/08

(54) **COLLAGEN PRODUCTION ENHANCER, PHARMACEUTICAL, COSMETIC, AND METHOD FOR PRODUCING COLLAGEN PRODUCTION ENHANCER**

(30) Priority: 25.09.2019 JP 2019174008
(71) Applicant: Murata Manufacturing Co., Ltd., Nagaokakyo-shi, Kyoto 617-8555 (JP)
(72) Inventor: SUNAHARA, Hirofumi, Nagaokakyo-shi, Kyoto 617-8555 (JP); YAMADA, Takaaki, Nagaokakyo-shi, Kyoto 617-8555 (JP)
(74) Representative: Reeve, Nicholas Edward
(86) International application number: PCT/JP2020/034171
(87) International publication number: WO 2021/059986

(57) **Abstract**

An agent for promoting collagen production, comprising a fermentation product of a Lamiaceae plant.

## Description

### TECHNICAL FIELD

The present invention relates to an agent for promoting collagen production, a medicament, a cosmetic, and a method for manufacturing an agent for promoting collagen production.

### BACKGROUND ART

In recent years, researches on the structure of human skin and the mechanism of its metabolism have progressed, which has gradually revealed the causes and mechanisms of changes in human skin with aging, such as wrinkles, fine wrinkles, blotches, and sagging. The skin is composed of an outer thin epidermis (epithelial tissue) and a thick dermis (connective tissue), which underlies the epidermis. The epidermis as the outmost layer of a living body protects the body against the external environment and prevents leakages of internal moisture and nutrients. The dermis is a connective tissue mainly composed of, for example, fibroblasts, collagen fibers (collagen), elastic fibers (elastin), and proteoglycans, which constitute a composite structure that spreads three-dimensionally, and plays a role in providing strength, stretchability, and elasticity to the skin. When the amount of sebum and moisture in the skin decreases with aging, the moisture retention capability of the stratum corneum on the skin is lost, which tends to result in small wrinkles and rough dry skin caused by drying. Under the circumstances, it has been proposed to improve the skin functions using a fermented product (see, e.g., Patent Documents 1 to 7).

### PRIOR ART DOCUMENTS

### PATENT DOCUMENTS

Patent Document 1: WO 2018/123828
Patent Document 2: Japanese Patent No. 5468183
Patent Document 3: Japanese Patent No. 2015-156832
Patent Document 4: Japanese Patent No. 5467106
Patent Document 5: Japanese Patent No. 4990297
Patent Document 6: Japanese Patent No. 2009-249366
Patent Document 7: Japanese Patent No. 2009-249365

### SUMMARY OF THE INVENTION

### Problem to be solved by the invention

An object of the present invention is to provide an agent for promoting collagen production, a medicament, and a cosmetic that comprise a novel component derived from a naturally occurring substance as an active ingredient, and a method for manufacturing an agent for promoting collagen production.

### Means for solving the problem

An agent for promoting collagen production according to one aspect of the present invention includes a fermentation product of a Lamiaceae plant. A medicament according to one aspect of the present invention includes a fermentation product of a Lamiaceae plant. A cosmetic according to one aspect of the present invention includes a fermentation product of a Lamiaceae plant. A method for manufacturing an agent for promoting collagen production according to one aspect of the present invention includes fermenting a Lamiaceae plant.

### Advantageous effect of the invention

According to the present invention, an agent for promoting collagen production, a medicament, and a cosmetic that comprise a novel component derived from a naturally occurring substance as an active ingredient, and a method for manufacturing an agent for promoting collagen production can be provided.

### BRIEF EXPLANATION OF THE DRAWINGS

FIG. 1 is a table showing the amount of collagen produced according to Example 3.
FIG. 2 is a table showing the results of an antibacterial test according to Example 4.
FIG. 3 is a table showing the results of an antiviral test according to Example 5.

### MODE FOR CARRYING OUT THE INVENTION

Embodiments of the present invention are described in detail below. It should be noted that the following embodiments are provided for illustrating apparatuses and methods for embodying the technical ideas of the present invention, and the technical ideas of the present invention do not limit combinations of components or the like to those provided below. The technical ideas of the present invention allow various changes within the scope of the claims.

The agent for promoting collagen production according to the embodiments includes a fermentation product of a Lamiaceae plant. Examples of the Lamiaceae or Labiatae plant include Isodon or Rabdosia plants. Examples of the Isodon plant include Isodon japonicus HARA or Rabdosia japonica HARA. The Isodon japonicus HARA is also referred to as Isodonis Herba.

The agent for promoting collagen production according to the embodiments may include a lactic acid bacterium derived from a Lamiaceae plant. The lactic acid bacteria are, for example, Lactobacillus bacteria. The Lactobacillus bacteria are a species belonging to the genus Lactobacillus and is a Gram-positive facultative anaerobe. The Lactobacillus bacteria ferment saccharides to produce lactic acid. Although bacteria belonging to the genus Lactobacillus also reside in the body of an animal including a human, the Lactobacillus bacteria according to the embodiments are Lactobacillus bacteria derived from a Lamiaceae plant. For example, the Lactobacillus bacteria according to the embodiments are extracted after fermenting a Lamiaceae plant.

Examples of the Lactobacillus bacteria derived from a Lamiaceae plant include L. parafarraginis, L. parabuchneri, L. buchneri, and L. harbinensis. Alternatively, examples of the Lactobacillus bacteria derived from a Lamiaceae plant include L. vini and L. nagelii. The agent for promoting collagen production according to the embodiments may include a plurality of species belonging to the genus Lactobacillus.

The agent for promoting collagen production according to the embodiments promotes collagen production of cells. Examples of the collagen include type I collagen. The type I collagen gives firmness, elasticity, and strength to skin. Decrease in type I collagen becomes a cause of wrinkles and aging. Further, type I collagen is involved in wound healing. Type I collagen consists of two α1 chains and one α2 chain, wherein the α1 chain is expressed from the COL1A1 gene and the α2 chain is expressed from the COL1A2 gene.

The agent for promoting collagen production according to the embodiments can be used as a medicament, a cosmetic, and/or a food for giving at least one of firmness, elasticity, and strength to skin. Further, the agent for promoting collagen production according to the embodiments can be used as a medicament, a cosmetic, and/or a food for improving an anti-wrinkle function. The agent for promoting collagen production according to the embodiments can be used as an anti-wrinkle medicament, an anti-aging medicament, an anti-wrinkle cosmetic, and an anti-aging cosmetic. Furthermore, the agent for promoting collagen production according to the embodiments can be used as a medicament, a cosmetic, and/or a food for wound healing. It should be noted that the wrinkles encompass fine wrinkles.

Further, the agent for promoting collagen production according to the embodiments also has a function of reducing microorganisms. Examples of the microorganism include bacteria and viruses. Examples of the bacteria include Gram-negative bacteria and Gram-positive bacteria.

The agent for promoting collagen production according to the embodiments, for example, reduces Gram-negative bacteria and Gram-positive bacteria by 80% or more, 85% or more, 90% or more, or 95% or more within 24 hours. Examples of the Gram-negative bacteria include, but are not limited to, Escherichia coli, Salmonella enterica, Vibrio parahaemolyticus, Klebsiella pneumoniae, and Pseudomonas aeruginosa. Examples of the Gram-positive bacteria include, but are not limited to, Staphylococcus aureus, methicillin-resistant Staphylococcus aureus (MRSA), spore-forming Bacillus cereus, and Bacillus subtilis.

The agent for promoting collagen production according to the embodiments also has a function of reducing viruses. The agent for promoting collagen production according to the embodiments, for example, reduces viruses by 80% or more, 85% or more, 90% or more, or 95% or more within 24 hours. The viruses include enveloped viruses, each of which is a virus having an envelope, and non-enveloped viruses, each of which is a virus lacking an envelope. Further, the enveloped viruses include DNA viruses and RNA viruses.

Examples of the DNA virus having an envelope include, but are not limited to, human herpes virus, vaccinia virus, and hepatitis B virus.

Examples of the RNA virus having an envelope include, but are not limited to, influenza virus, SARS coronavirus, RS virus, mumps virus, Lassa virus, dengue virus, rubella virus, human immunodeficiency virus, measles virus, hepatitis C virus, Ebola virus, yellow fever virus, and Japanese encephalitis virus.

Examples of the DNA virus lacking an envelope include, but are not limited to, adenovirus, B19 virus, papovavirus, and human papilloma virus.

Examples of the RNA virus lacking an envelope include, but are not limited to, norovirus, calicivirus, poliovirus, echovirus, hepatitis A virus, hepatitis E virus, rhinovirus, astrovirus, rotavirus, Coxsackie virus, enterovirus, and sapovirus.

The agent for promoting collagen production according to the embodiments contains an effective amount of a fermentation product of a Lamiaceae plant. The agent for promoting collagen production according to the embodiments may contain the fermentation product of a Lamiaceae plant in a solvent such as water or in a fermentation liquid of a plant. Examples of the plant from which the fermentation liquid is obtained include a Lamiaceae plant as in the Lactobacillus bacteria. The effective amount refers to an amount required for promoting collagen production of cells, and is appropriately determined depending on, for example, the age, the type, and the site applied of a human or animal as a subject.

The lactic acid bacteria contained in the agent for promoting collagen production according to the embodiments may be live bacteria, or may be dead bacteria subjected to, for example, a heat treatment. Accordingly, the agent for promoting collagen production according to the embodiments may contain dead bacteria of lactic acid bacteria. The lactic acid bacteria may be a dried bacterial product. The dead bacteria of lactic acid bacteria or the dried bacterial product also exerts an effect of promoting collagen production. Further, the dead bacteria of lactic acid bacteria or the dried bacterial product is easy to transport and store for a long time.

The agent for promoting collagen production according to the embodiments can be, for example, a liquid, a cream, an ointment, a plaster, a gel, a wax, and a spray.

The agent for promoting collagen production according to the embodiments can be, for example, a skin conditioning cosmetic. Examples of the skin conditioning cosmetic include a lotion, an essence, and a pack. The agent for promoting collagen production according to the embodiments can be, for example, a barrier cosmetic. Examples of the barrier cosmetic include a barrier emulsion and a barrier cream. The agent for promoting collagen production according to the embodiments can be, for example, a base makeup cosmetic. Examples of the base makeup cosmetic include a foundation, white makeup powder, and a foundation primer. The agent for promoting collagen production according to the embodiments can be, for example, a point makeup cosmetic. Examples of the point makeup cosmetic include a lipstick, an eye makeup, a cheek, and a nail enamel.

Further, the agent for promoting collagen production according to the embodiments is provided as, for example, a disinfectant, a dermatological agent such as a therapeutic ointment, an eye drop, and an oral medicine. The agent for promoting collagen production according to the embodiments is administered to, for example, skin of human body including a hand, a foot, and a finger, a hair, an oral cavity, and an eye.

The agent for promoting collagen production according to the embodiments can contain, in addition to the fermentation product of a Lamiaceae plant, compounding ingredients of a cosmetic and a medicament, such as a liquid oil and fat, a solid oil and fat, a wax, a hydrocarbon, a higher fatty acid, a higher alcohol, an ester, silicone, an anionic surfactant, a cationic surfactant, an amphoteric surfactant, a non-ionic surfactant, a moisturizing agent, a water-soluble polymer, a thickening agent, a coating agent, a metal ion sequestering agent, a lower alcohol, a polyhydric alcohol, a saccharide, an amino acid, an organic amine, a pH adjusting agent, a skin nutrient, a vitamin, an antioxidant, a flavoring agent, a powdery substance, a coloring material, and water, as appropriate, depending on the intended purpose.

When the agent for promoting collagen production according to the embodiments contains an oily component, the concentration of the oily component in the agent for promoting collagen production according to the embodiments is not particularly limited, and examples include 0.1% or more by mass and 90% or less by mass, or 0.5% or more by mass and 90% or less by mass. When the agent for promoting collagen production according to the embodiments contains an aqueous component, the concentration of the aqueous component in the agent for promoting collagen production according to the embodiments is not particularly limited, and examples include 0.1% or more by mass and 90% or less by mass, or 0.5% or more by mass and 90% or less by mass. The ratio between the oily component and the aqueous component in the agent for promoting collagen production according to the embodiments is appropriately determined depending on whether the agent for promoting collagen production according to the embodiments is an oil-in-water (O/W) agent or a water-in-oil (W/O) agent. When the agent for promoting collagen production according to the embodiments contains a surfactant, the concentration of the surfactant in the agent for promoting collagen production according to the embodiments is not particularly limited, and examples include 2% or more by mass and 10% or less by mass.

The agent for promoting collagen production according to the embodiments is manufactured by fermenting a Lamiaceae plant to obtain a fermentation product. When the Lamiaceae plant is fermented, salt and saccharides such as molasses are added to the Lamiaceae plant. The fermentation temperature is, for example, 30°C. The hydrogen ion exponent (pH) of the resulting fermentation liquid is around 4.0. Materials secreted by the Lactobacillus bacteria can be extracted from the fermentation liquid.

The resulting fermentation liquid may be heated to kill Lactobacillus bacteria contained in the fermentation liquid to obtain dead bacteria. Accordingly, the fermentation liquid can be a heat-treated fermentation liquid. Further, the fermentation liquid may be spray-dried to obtain a dried bacterial product of the Lactobacillus bacteria. The dried bacterial product can also be prepared by, for example, a freeze-drying method and a hot-air drying method.

Furthermore, the resulting fermentation liquid, bacterial cells of the Lactobacillus bacteria, or the dried bacterial product of the Lactobacillus bacteria may be added to soy milk, and the soy milk is fermented to obtain a soy milk fermentation liquid. The soy milk fermentation liquid also exerts the effect as an agent for promoting collagen production.

As described above, the agent for promoting collagen production or the like according to each of the embodiments of the present invention has the configuration and the action and effect in the following examples according to any one or a combination of two or more of those described above.

The agent for promoting collagen production according to the present embodiments includes a fermentation product of a Lamiaceae plant. In the agent for promoting collagen production according to the present embodiments, the Lamiaceae plant may be an Isodon plant. In the agent for promoting collagen production according to the present embodiments, the Isodon plant may be Isodon japonicus HARA. In the agent for promoting collagen production according to the present embodiments, the Isodon plant may be Isodonis Herba.

The agent for promoting collagen production according to the present embodiments may promote expression of type I collagen in a cell.

The agent for promoting collagen production according to the present embodiments may include lactic acid bacteria derived from a Lamiaceae plant. In the agent for promoting collagen production according to the present embodiments, the lactic acid bacteria may include Lactobacillus bacteria. In the agent for promoting collagen production according to the present embodiments, the species of the Lactobacillus bacteria may be at least one selected from the group consisting of L. parafarraginis, L. parabuchneri, L. buchneri, L. harbinensis, L. vini, and L. nagelii.

In the agent for promoting collagen production according to the present embodiments, the lactic acid bacteria may be live bacteria, or may be dead bacteria. In the agent for promoting collagen production according to the present embodiments, the lactic acid bacteria may be subjected to a heat treatment. In the agent for promoting collagen production according to the present embodiments, the lactic acid bacteria may be a dried bacterial product.

The medicament according to the present embodiments includes a fermentation product of a Lamiaceae plant. In the medicament according to the present embodiments, the Lamiaceae plant may be an Isodon plant. In the medicament according to the present embodiments, the Isodon plant may be Isodon japonicus HARA. In the medicament according to the present embodiments, the Isodon plant may be Isodonis Herba.

The medicament according to the present embodiments may include lactic acid bacteria derived from a Lamiaceae plant. In the medicament according to the present embodiments, the lactic acid bacteria may include Lactobacillus bacteria. In the medicament according to the present embodiments, the species of the Lactobacillus bacteria may be at least one selected from the group consisting of L. parafarraginis, L. parabuchneri, L. buchneri, L. harbinensis, L. vini, and L. nagelii.

In the medicament according to the present embodiments, the lactic acid bacteria may be live bacteria, or may be dead bacteria. In the medicament according to the present embodiments, the lactic acid bacteria may be subjected to a heat treatment. In the medicament according to the present embodiments, the lactic acid bacteria may be a dried bacterial product.

The medicament according to the present embodiments may promote expression of collagen in a cell. The medicament according to the present embodiments may promote expression of type I collagen in a cell.

The medicament according to the present embodiments may be an anti-wrinkle medicament. The medicament according to the present embodiments may be an anti-aging medicament.

The cosmetic according to the present embodiments includes a fermentation product of a Lamiaceae plant. In the cosmetic according to the present embodiments, the Lamiaceae plant may be an Isodon plant. In the cosmetic according to the present embodiments, the Isodon plant may be Isodon japonicus HARA. In the cosmetic according to the present embodiments, the Isodon plant may be Isodonis Herba.

The cosmetic according to the present embodiments may include lactic acid bacteria derived from a Lamiaceae plant. In the cosmetic according to the present embodiments, the lactic acid bacteria may include Lactobacillus bacteria. In the cosmetic according to the present embodiments, the species of the Lactobacillus bacteria may be at least one selected from the group consisting of L. parafarraginis, L. parabuchneri, L. buchneri, L. harbinensis, L. vini, and L. nagelii.

In the cosmetic according to the present embodiments, the lactic acid bacteria may be live bacteria, or may be dead bacteria. In the cosmetic according to the present embodiments, the lactic acid bacteria may be subjected to a heat treatment. In the cosmetic according to the present embodiments, the lactic acid bacteria may be a dried bacterial product.

The cosmetic according to the present embodiments may promote expression of collagen in a cell. The cosmetic according to the present embodiments may promote expression of type I collagen in a cell.

The cosmetic according to the present embodiments may be an anti-wrinkle cosmetic. The cosmetic according to the present embodiments may be an anti-aging cosmetic.

In the method for manufacturing an agent for promoting collagen production according to the present embodiments, the manufacturing method includes fermenting a Lamiaceae plant. In the method for manufacturing an agent for promoting collagen production according to the present embodiments, the Lamiaceae plant may be an Isodon plant. In the method for manufacturing an agent for promoting collagen production according to the present embodiments, the Isodon plant may be Isodon japonicus HARA. In the method for manufacturing an agent for promoting collagen production according to the present embodiments, the Isodon plant may be Isodonis Herba.

The method for manufacturing an agent for promoting collagen production according to the present embodiments may include obtaining lactic acid bacteria derived from a Lamiaceae plant. In the method for manufacturing an agent for promoting collagen production according to the present embodiments, the lactic acid bacteria may include Lactobacillus bacteria. In the method for manufacturing an agent for promoting collagen production according to the present embodiments, the species of the Lactobacillus bacteria may be at least one selected from the group consisting of L. parafarraginis, L. parabuchneri, L. buchneri, L. harbinensis, L. vini, and L. nagelii.

In the method for manufacturing an agent for promoting collagen production according to the present embodiments, lactic acid bacteria may be obtained as live bacteria, or lactic acid bacteria may be obtained as dead bacteria. In the method for manufacturing an agent for promoting collagen production according to the present embodiments, the lactic acid bacteria may be subjected to a heat treatment. In the method for manufacturing an agent for promoting collagen production according to the present embodiments, the lactic acid bacteria may be made into a dried bacterial product.

### EXAMPLES

Examples of the present invention are described below. However, needless to say, the present invention is not limited to the following examples.

### (Example 1: Lamiaceae plant fermentation product)

In a Lamiaceae plant, it is thought that the number of lactic acid bacteria reaches a maximum between one hour before and after sunrise (i.e., in a total of 2 hours) in a day. Further, it is thought that lactic acid bacteria decrease and photosynthetic bacteria increase outside of the time period. Thus, during this time period of 2 hours, end portions of Isodonis Herba each having a length of about 20 cm were harvested. Immediately, 6.3 kg of the harvested Isodonis Herba was placed in a first pickle barrel with a plastic bag lining, the Isodonis Herba was sprinkled with 3.2 kg of molasses and 0.6 kg of coarse salt, and thereafter the opening of the plastic bag was closed and hermetically sealed. A stone weight was put on the plastic bag to pickle the Isodonis Herba.

Several days after the time when the pickle juice rose above the Isodonis Herba, the stone weight was removed. Next, 10 L of chlorine-free water for rinsing was poured into a second pickle barrel, and the pickled Isodonis Herba was immersed in and 10 kg of the pickle juice was added to the water. Further, a third pickle barrel was prepared, and a wire mesh filter was placed on the opening of the third pickle barrel. The Isodonis Herba was retrieved with hand-washing by rubbing from the second pickle barrel piece by piece, and the Isodonis Herba was gently pressed against a wire mesh filter on the opening of a third pickle barrel by the palm to squeeze pickle juice.

After squeezing all of the Isodonis Herba, the pickle juice remaining in the second juice of pickle was passed through a wire mesh filter for filtration. Next, in the pickle juice in the third pickle barrel, molasses (Hateruma brown sugar) was added and dissolved to give a final concentration of 10 wt%, and coarse salt was added and dissolved to give a final concentration of 3 wt%. Thereafter, fermentation was started in the third pickle barrel at an ambient temperature of about 30°C. It was observed that initially large bubbles formed, gradually the size of bubble formed decreased, and finally the bubble formation ceased. The bubble formation ceased after about 1 week, and the pH was about 3.8 at this time. The pickle juice at this time was used as an Isodonis Herba fermentation liquid. A portion of the obtained Isodonis Herba fermentation liquid was heated at 70°C for 30 minutes to kill bacteria such as lactic acid bacteria and obtain a heat-treated Isodonis Herba fermentation liquid.

### (Example 2: Soy milk fermentation liquid using Lamiaceae plant fermentation product)

Soy milk was heated to 70°C to undergo superheating sterilization treatment for about 30 minutes. To the soy milk subjected to the heat sterilization treatment, the heat-untreated Isodonis Herba fermentation liquid prepared in Example 1 was added to give a final concentration of about 10 wt%, and the mixture was sufficiently stirred. Subsequently, the soy milk containing the heat-untreated Isodonis Herba fermentation liquid was fermented at 37°C for 24 hours. After fermentation, solid contents were removed by filtration to obtain a soy milk fermentation liquid containing the Isodonis Herba fermentation liquid.

### (Example 3: Promoting collagen production by Lamiaceae plant fermentation product)

Normal human dermal fibroblasts were maintained using a DMEM medium (+5% FBS) in a culture vessel to confluence. After reaching confluence, the medium was removed from the culture vessel, DMEM (0% FBS) containing 600 µmol/L of hydrogen peroxide (H₂O₂) was added to the culture vessel, and the cells were cultured at 37°C for 1 hour. After cultured for 1 hour, the H₂O₂-containing DMEM (0% FBS) was removed from the culture vessel, and a DMEM medium (+10% FBS) was added to the culture vessel. After this procedure was repeated once daily for 4 days, the cells were further cultured in DMEM (10% FBS) for 3 days, and the cultured cells were obtained and used as aging induction-treated cells.

The aging induction of the cells was confirmed by senescence-associated beta-galactosidase (SA-β-Gal, an aging marker) staining.

The aging induction-treated cells were seeded in 48-well plate at a cell density of 5.0 × 10⁴ cells/well using a DMEM medium (+5% FBS). At 24 hours post-seeding, the medium in each well was replaced with one of the following media: a DMEM medium (+0.5% FBS) containing the heat-untreated Isodonis Herba fermentation liquid prepared in Example 1 at a concentration of 1.0% or 10.0%, a DMEM medium (+0.5% FBS) containing Vitamin C magnesium phosphate at a concentration of 25 µmol/L, a DMEM medium (+0.5% FBS) containing vitamin C at a concentration of 25 µmol/L, and a DMEM medium (+0.5% FBS). Then, the cells were cultured for 48 hours, thereafter the medium was collected, and the amount of type I collagen in the medium was quantified by an ELISA method (direct ELISA using an anti-Human Collagen Type I antibody (Rabbit)).

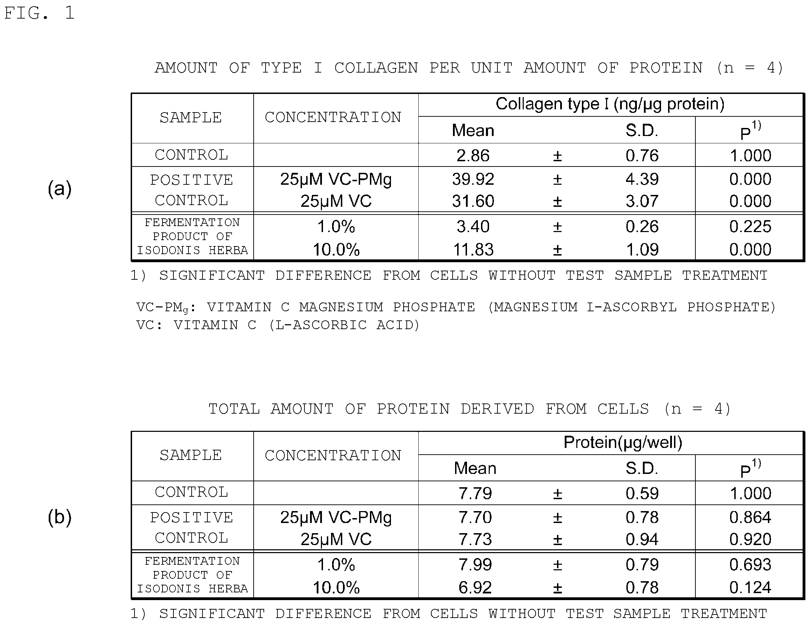

As a result, as shown in FIG. 1, it was shown that when the cells were cultured in the medium containing Vitamin C magnesium phosphate or vitamin C (positive control), the production of type I collagen was promoted. Further, it was also shown that when the cells were cultured in the medium to which the Isodonis Herba fermentation liquid was added, the production of type I collagen was also promoted.

### (Example 4: Antimicrobial effect of Lamiaceae plant fermentation product)

Escherichia coli, Salmonella enterica, Vibrio parahaemolyticus, and Klebsiella pneumoniae were prepared as Gram-negative cocci, and Pseudomonas aeruginosa was prepared as Gram-negative bacillus. Further, Staphylococcus aureus and MRSA were prepared as Gram-positive cocci, and Bacillus subtilis and Bacillus cereus were prepared as Gram-positive bacillus.

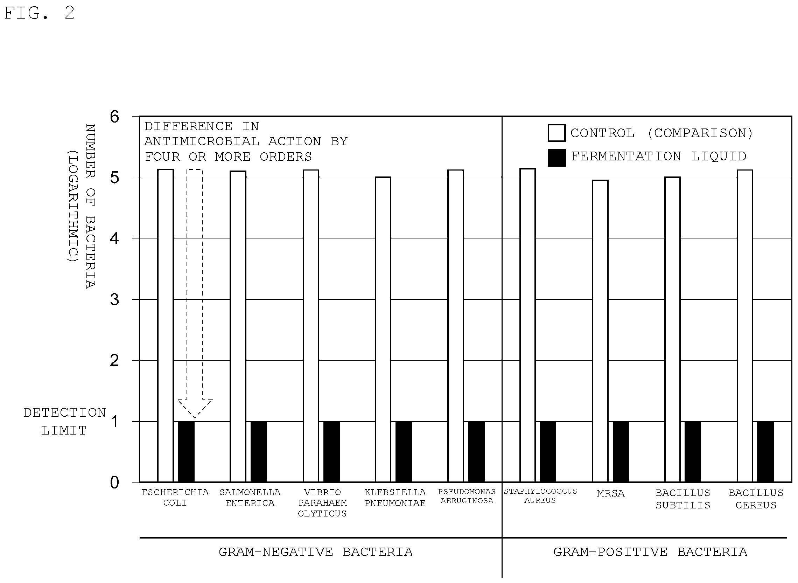

In 10 mL of the heat-untreated Isodonis Herba fermentation liquid prepared in Example 1, 0.1 mL of the bacterial suspension containing any one of the above-described bacteria at a concentration of 10⁷ cells/mL was seeded and allowed to be affected by the fermentation liquid at 25°C, and the number of bacteria seeded was counted over time for 24 hours. Control experiments were performed in the same way except that phosphate buffer (1/15 mol/L, pH 7.2) was used instead of the Isodonis Herba fermentation liquid. As a result, as shown in FIG. 2, the Isodonis Herba fermentation liquid reduced all types of the prepared bacteria within 24 hours. It should be noted that when the soy milk fermentation liquid containing the Isodonis Herba fermentation liquid prepared in Example 2 was used, the same results were obtained.

### (Example 5: Antiviral effect of Lamiaceae plant fermentation product)

A virus growth medium in which influenza virus type A (H1N1) as an enveloped virus was grown was prepared. Further, a virus growth medium in which feline calicivirus, which was a virus alternative to norovirus, as a non-enveloped virus was grown was prepared. The virus growth medium was serially diluted 10-fold with purified water. Thereafter, according to 50% tissue culture infectious dose (TCID 50), an antiviral test with the heat-untreated Isodonis Herba fermentation liquid prepared in Example 1 was performed at room temperature. The antiviral test was performed in Japan Food Research Laboratories.

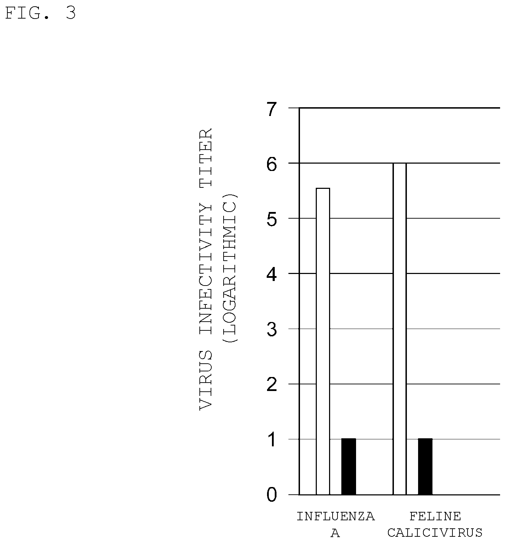

As a result, as shown in FIG. 3, the Isodonis Herba fermentation liquid reduced infectivity titer of influenza virus and feline calicivirus within 1 hour. It should be noted that when the soy milk fermentation liquid containing the Isodonis Herba fermentation liquid prepared in Example 2 was used, the same results were obtained.

The above-described embodiments and examples are provided to facilitate the understanding of the present invention, and it is not intended that the present invention is limited thereto. The present invention can be changed/improved without departing from the spirit of the present invention, and the present invention also includes equivalents thereof. That is, when a change in design of each of the embodiments and examples is properly made by those of ordinary skill in the art, the changed embodiment or example is also encompassed within the scope of the present invention as long as it has features of the present invention. For example, each element or the like of the embodiments and examples is not limited to those exemplified herein, and can be suitably changed. Further, needless to say, the embodiments and examples are illustrative, elements shown in different embodiments can be replaced or combined in part, and the replaced or combined embodiment is also encompassed within the scope of the present invention as long as it has features of the present invention.

## Claims

1. An agent for promoting collagen production, comprising a fermentation product of a Lamiaceae plant.

2. The agent for promoting collagen production according to claim 1, wherein the Lamiaceae plant is an Isodon plant.

3. The agent for promoting collagen production according to claim 2, wherein the Isodon plant is Isodon japonicus HARA.

4. The agent for promoting collagen production according to claim 2, wherein the Isodon plant is Isodonis Herba.

5. The agent for promoting collagen production according to any one of claims 1 to 4, wherein the collagen is type I collagen.

6. The agent for promoting collagen production according to any one of claims 1 to 5, comprising lactic acid bacteria derived from the Lamiaceae plant.

7. The agent for promoting collagen production according to claim 6, wherein the lactic acid bacteria include Lactobacillus bacteria.

8. The agent for promoting collagen production according to claim 7, wherein a species of the Lactobacillus bacteria is at least one selected from the group consisting of L. parafarraginis, L. parabuchneri, L. buchneri, L. harbinensis, L. vini, and L. nagelii.

9. The agent for promoting collagen production according to any one of claims 6 to 8, wherein the lactic acid bacteria are dead bacteria.

10. The agent for promoting collagen production according to any one of claims 6 to 9, wherein the lactic acid bacteria are subjected to a heat treatment.

11. The agent for promoting collagen production according to any one of claims 6 to 10, wherein the lactic acid bacteria are a dried bacterial product.

12. A medicament comprising the agent for promoting collagen production according to any one of claims 1 to 11.

13. The medicament according to claim 12, wherein the medicament is an anti-wrinkle medicament.

14. The medicament according to claim 12, wherein the medicament is an anti-aging medicament.

15. A cosmetic comprising the agent for promoting collagen production according to any one of claims 1 to 11.

16. The cosmetic according to claim 15, wherein the cosmetic is an anti-wrinkle cosmetic.

17. The cosmetic according to claim 15, wherein the cosmetic is an anti-aging cosmetic.

18. A method for manufacturing an agent for promoting collagen production, comprising fermenting a Lamiaceae plant.

19. The method for manufacturing an agent for promoting collagen production according to claim 18, wherein the Lamiaceae plant is an Isodon plant.

20. The method for manufacturing an agent for promoting collagen production according to claim 19, wherein the Isodon plant is Isodon japonicus HARA.

21. The method for manufacturing an agent for promoting collagen production according to claim 19, wherein the Isodon plant is Isodonis Herba.
